(19) Europäisches Patentamt
European Patent Office
Office européen des brevets

(11) EP 0 753 295 B1

(12) FASCICULE DE BREVET EUROPEEN

(45) Date de publication et mention
de la délivrance du brevet:
**08.04.1998 Bulletin 1998/15**

(51) Int. Cl.$^6$: **A61K 7/42**, A61K 7/00,
A61K 7/48

(21) Numéro de dépôt: **96401430.2**

(22) Date de dépôt: **27.06.1996**

(54) **Utilisation de particules creuses déformables contre le photobleuissement et/ou le blanchiment d'une composition cosmétique et/ou dermatologique contenant des pigments d'oxyde de titane**

Verwendung von hohlen verformbaren Partikeln gegen die Photoverbläuung und/oder das Bleichen eines kosmetischen und/oder dermatologischen Mittels, das Titandioxidpigmente enthält

Use of deformable hollow particles against the photoblueing and/or the whitening of a cosmetic and/or dermatologic composition containing titanium oxide pigments

(84) Etats contractants désignés:
**AT BE CH DE ES FR GB IT LI NL SE**

(30) Priorité: **13.07.1995 FR 9508564**
**13.07.1995 FR 9508565**

(43) Date de publication de la demande:
**15.01.1997 Bulletin 1997/03**

(73) Titulaire: **L'OREAL**
**75008 Paris (FR)**

(72) Inventeur: **Hansenne, Isabelle**
**75017 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**DE-B- 2 521 003**        **FR-A- 2 700 952**
**GB-A- 2 191 945**        **US-A- 5 314 683**

- **CHEMICAL ABSTRACTS, vol. 104, no. 4, 27 Janvier 1986 Columbus, Ohio, US; abstract no. 24076w, page 290; XP002015534 & JP-A-60 184 004 (POLA CHEMICAL INDUSTRIES INC.) 19 Septembre 1985**
- **PATENT ABSTRACTS OF JAPAN vol. 11, no. 18 (C-398) [2465] , 17 Janvier 1987 & JP-A-61 194013 (TAISHO PHARMACEUT. CO LTD), 28 Août 1986,**

**Description**

L'invention se rapporte à l'utilisation de particules creuses déformables particulières dans une composition cosmétique et/ou dermatologique contenant des nanopigments d'oxyde de titane, afin de réduire le photobleuissement dû à la présence desdits pigments et/ou le blanchiment habituellement observé lors de l'application sur la peau de ce type de composition, ces particules creuses présentant une granulométrie allant de 1 μm à 250 μm, et étant constituées de copolymère de chlorure de vinylidène, de (méth)acrylate et d'acrylonitrile.

Les compositions selon l'invention peuvent en particulier être utilisées pour la photoprotection de la peau et des cheveux sous la forme de compositions antisolaires, mais également pour le maquillage, le soin, l'hygiène de la peau, du visage, du corps humain y compris le cuir chevelu et les muqueuses, des cheveux et enfin pour le traitement thérapeutique de la peau et des muqueuses. Ainsi, la composition à laquelle s'applique l'invention peut être une émulsion huile-dans-eau, une émulsion eau-dans-huile, une crème de soin, un baume, un blush ou un fond de teint fluide ou coulé, un onguent dermopharmaceutique.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Pour filtrer les rayonnements UV-A et UV-B, il existe sur le marché différents types de filtres solaires : les pigments minéraux et les filtres organiques. Ces filtres doivent pouvoir absorber ou bloquer les rayons nocifs du soleil tout en restant inoffensifs pour l'utilisateur.

Ainsi, de nombreux filtres organiques solaires capables d'absorber plus ou moins sélectivement les rayons UV nocifs ont été proposés à ce jour dans le domaine de la cosmétique. Pour diverses raisons, ces filtres ne donnent cependant pas entièrement satisfaction.

C'est pourquoi on cherche de plus en plus à éviter l'emploi de ces filtres organiques en privilégiant l'utilisation des pigments minéraux, qui jouent également le rôle de filtres solaires, principalement par diffusion / réflexion des UV, tout en apportant une sécurité plus grande pour l'utilisateur.

A ce titre, le pigment minéral le plus courant utilisé à ce jour est l'oxyde de titane dont les propriétés filtrantes sont bien connues.

Il est connu du document FR-A-2 700 952 des gels dans lesquels on a introduit des microsphères creuses particulières afin d'en améliorer les propriétés cosmétiques; ces gels peuvent également comprendre de l'oxyde de titane. Il est également connu du document GB-A-0 191 945 des poudres libres pour le maquillage dans lesquelles on a introduit des microsphères creuses particulières pour améliorer leurs propriétés cosmétiques; ces poudres peuvent également comprendre de l'oxyde de titane.

Toutefois, il est nécessaire, afin d'obtenir des facteurs de protection convenables, de pouvoir introduire dans les compositions solaires des pourcentages élevés en $TiO_2$, en particulier supérieurs à 5% en poids.

Mais, avec de telles teneurs en $TiO_2$, on observe d'une part, une instabilité à la lumière des compositions contenant ces pigments dans un milieu sans oxygène, qui se manifeste par l'apparition d'une coloration bleue (photocoloration connue sous le nom de photobleuissement) et d'autre part, l'apparition d'un blanchiment lorsqu'on applique ces compositions sur la peau. Ces deux phénomènes ne sont évidemment pas souhaitables d'un point de vue esthétique.

La présente invention vise à résoudre les problèmes énoncés ci-dessus.

Ainsi, à la suite d'importantes recherches menées dans le domaine de la photoprotection évoqué ci-dessus, la Demanderesse a maintenant découvert que l'introduction de particules creuses déformables de masse volumique et de granulométrie particulières dans une composition contenant des pigments de $TiO_2$ réduisait significativement les phénomènes de photobleuissement et de blanchiment intrinsèquement attachés à cette dernière.

Cette découverte est à la base de la présente invention.

En plus de la réduction des phénomènes de photobleuissement et de blanchiment, ces particules confèrent également de la douceur et de l'homogénéité à l'application ainsi qu'une plus grande facilité d'étalement.

De façon plus précise, l'invention se rapporte donc à l'utilisation de particules creuses déformables, dans une composition cosmétique et/ou dermatologique contenant des pigments de $TiO_2$, pour diminuer ou éliminer le photobleuissement dû à la présence desdits pigments et/ou le blanchiment lors de l'application de ladite composition sur la peau, ces particules creuses ayant une granulométrie allant de 1 μm à 250 μm et étant formées d'un copolymère de chlorure de vinylidène, d'acrylonitrile et de (méth)acrylate de méthyle, expansé.

L'invention a aussi pour objet un procédé pour réduire ou éliminer le photobleuissement et/ou le blanchiment lors de l'application sur la peau d'une composition cosmétique et/ou dermatologique contenant des pigments d'oxyde de titane, consistant à introduire dans la composition des particules creuses déformables présentant une granulométrie allant de 1 µm à 250 µm et étant formées d'un copolymère de chlorure de vinylidène, d'acrylonitrile et de (méth)acrylate de méthyle.

Par" déformable", il faut comprendre que les particules sont souples et élastiques : après écrasement, elles reprennent leur forme initiale.

Les particules ont généralement une granulométrie allant de 1 µm à 250 µm.

De façon avantageuse, les particules ont une granulométrie inférieure à 100 µm. En effet, plus les particules sont fines, plus la composition est douce à l'application. De préférence, les particules ont une granulométrie allant de 10 µm à 60 µm.

De façon avantageuse, les particules ont une masse volumique allant de 15 kg/m$^3$ à 200 kg/m$^3$ et mieux supérieure à 40 kg/m$^3$ et/ou inférieure à 100 kg/m$^3$ et notamment allant de 60 kg/m$^3$ à 80 kg/m$^3$.

On peut, par exemple, utiliser un copolymère contenant : de 1 % à 60 % de motifs dérivés du chlorure de vinylidène, de 20 % à 90 % de motifs dérivés d'acrylonitrile et de 1 % à 50 % de motifs dérivés d'un monomère acrylique, la somme des pourcentages (en poids) étant égale à 100. Le monomère acrylique est par exemple un acrylate ou méthacrylate de méthyle et, en particulier le méthacrylate. Ces particules se présentent notamment à l'état sec ou hydraté.

Ce copolymère est non toxique et non irritant pour la peau.

De préférence, les particules se présentent sous forme de microsphères.

Les particules de l'invention peuvent être obtenues, par exemple, selon les procédés des brevets et demandes de brevet EP-56 219, EP-348 372, EP-486 080, EP-320 473, EP-112 807 et US-3 615 972.

La cavité interne des particules contient en principe un gaz qui peut être de l'air, de l'azote ou un hydrocarbure comme l'isobutane ou l'isopentane.

Les particules creuses utilisables dans l'invention sont en particulier celles vendues sous la marque Expancel par la Société Nobel Casco telles que l'Expancel 551 DE 20 de granulométrie de 30 µm et de masse volumique de 65 kg/m$^3$ environ ; l'Expancel 551 DE 50 de granulométrie de 40 µm.

Dans les compositions de l'invention, on utilise de préférence de 0,1 % à 10 % en poids de particules et mieux de 0,3 % à 5 % en poids et encore de 0,3 % à 2 %, par rapport au poids total de la composition.

Une des caractéristiques essentielles des compositions visées par la présente invention est de contenir des pigments d'oxyde de titane. Les pigments visés par la présente invention sont les pigments d'oxyde de titane connus, habituellement utilisés dans le domaine cosmétique comme charges ou comme filtres, qui peuvent être traités ou non traités. De tels pigments comprennent les nanopigments d'oxyde de titane. Par nanopigments, on entend des pigments dont la taille moyenne des particules élémentaires est comprise entre 5 et 100 nm.

L'oxyde de titane peut se présenter sous forme rutile, anatase ou amorphe, mais de préférence sous forme rutile et/ou anatase.

Les pigments traités peuvent par exemple être traités par l'alumine, la silice, les composés de l'aluminium, les composés du silicium, les composés du sodium, les oxydes de fer, les esters de fer, l'acide stéarique, la glycérine.

Plus particulièrement, les pigments traités peuvent être des oxydes de titane traités par:

- la silice et l'alumine tels que les produits « MICROTITANIUM DIOXIDE MT 500 SA » et « MICROTITANIUM DIOXIDE MT 100 SA » de la société TAYCA, et les produits « TIOVEIL Fin », « TIOVEIL OP », « TIOVEIL MOTG » et « TIOVEIL IPM » de la société TIOXIDE,
- l'alumine et le stéarate d'aluminium tels que le produit « MICROTITANIUM DIOXIDE MT 100 T » de la société TAYCA,
- l'alumine et le laurate d'aluminium tels que le produit « MICROTITANIUM DIOXIDE MT 100 S » de la société TAYCA,
- des oxydes de fer et le stéarate de fer tels que le produit « MICROTITANIUM DIOXIDE MT 100 F » de la société TAYCA,
- la silice, l'alumine et la silicone tels que les produits « MICROTITANIUM DIOXIDE MT 100 SAS », « MICROTITANIUM DIOXIDE MT 600 SAS » et « MICROTITANIUM DIOXIDE MT 500 SAS » de la société TAYCA,
- l'hexamétaphosphate de sodium tels que le produit « MICROTITANIUM DIOXIDE MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les oxydes de titane non traités peuvent par exemple être ceux vendus par la société TAYCA sous les dénominations commerciales « MICROTITANIUM DIOXIDE MT 500 B » ou « MICROTITANIUM DIOXIDE MT 600 B ».

Le ou les (nano)pigments d'oxyde de titane peuvent être présents dans la composition selon l'invention dans une proportion comprise entre 1 et 30% en poids par rapport au poids total de la composition, de préférence, entre 2 et 25% en poids par rapport au poids total de la composition.

Grâce à l'invention, on peut sans problème introduire des quantités relativement élevées de $TiO_2$, en particulier supérieures à 5% en poids, ou encore supérieures à 10% en poids, ou même supérieures à 15% en poids, et ceci sans être pénalisé par le problème du photobleuissement évoqué ci-avant.

Les autres constituants pouvant rentrer dans la formulation des compositions visées par l'invention, en particulier les huiles, les composés cireux, les épaississants, les émulsionnants, les gélifiants sont ceux qui sont classiquement utilisés dans le domaine cosmétique et/ou dermatologique.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer l'huile de jojoba, la paraffine, la cire de carnauba, la cire d'abeille, l'huile de ricin hydrogénée.

Comme émulsionnants, on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols, les tensioactifs anioniques (alkylphosphate de K ou de Na), les alcools gras polyalcoxylés.

De préférence, on utilise les alcools gras polyalcoxylés tels que les alcools butyliques oxypropylénés, les alcools capryliques oxyéthylénés, les alcools cétyliques oxyéthylénés.

Comme épaississants, on peut utiliser les acides polyacryliques réticulés, les gommes de guar et celluloses modifiées ou non telles que la gomme de guar hyd roxypropylée, la méthylhydroxyéthylcellulose, l'hydroxypropylméthyl cellulose ou encore l'hydroxyéthylcellulose.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

Les compositions visées par la présente invention peuvent aussi contenir divers ingrédients classiquement utilisés dans le domaine cosmétique, dermatologique ou dermopharmaceutique comme les matières colorantes et les pigments autres que les oxydes de titane, les solvants (eau, alcools,...), les conservateurs, les parfums, les actifs hydratants, les agents absorbant les rayons ultraviolets (filtres solaires, pigments minéraux autres que les oxydes de titane), les agents pulvérulents autres que les particules creuses déformables, des agents bactéricides et/ou des absorbeurs d'odeur.

Ces compositions peuvent, en outre, contenir un ou plusieurs actifs cosmétiques ou dermatologiques hydrophiles et mieux lipophiles, notamment en vue de traiter et/ou prévenir les affections cutanées telles que l'acné, les mycoses, l'eczéma, la rosacée, les dermites séborrhéïques, les hélio dermatoses, le vieillissement cutané ainsi que les affections du cuir chevelu. Ces compositions sont destinées au traitement de la peau par voie topique.

Les compositions concernées par l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

L'invention convient particulièrement bien au cas des compositions de type anhydre, en particulier les crèmes anhydres.

Des exemples concrets, mais nullement limitatifs, illustrant l'invention, vont maintenant être donnés.

On a réalisé des tests comparatifs afin de mettre en évidence l'amélioration apportée en ce qui concerne le photobleuissement et le blanchiment sur la peau par l'utilisation de microsphères creuses déformables d'un copolymère expansé de chlorure de vinylidène et d'acrylonitrile ou de chlorure de vinylidène, d'acrylonitrile et de méthacrylate, au sein d'une composition contenant des pigments d'oxyde de titane.

## EXEMPLE 1:

On a réalisé une composition anhydre contenant un certain pourcentage x d'Expancel et 20% d'oxyde de titane, dans un support anhydre composé d'un alcool gras polyalcoxylé (alcool butylique oxypropyléné), d'un corps gras de la classe des esters (palmitate d'isopropyle), d'une cire dont le point de fusion est compris entre 60 et 80°C (huile de ricin hydrogénée), d'un corps gras épaississant (monostéarate de glycérol) et d'une huile (polydécène hydrogéné).

On a ainsi réalisé cinq compositions différentes A, B, C, D et E en faisant varier la teneur x de 0 à 2%.

Pour ces cinq compositions, le photobleuissement a été évalué selon le protocole suivant : les compositions ont été introduites dans des boîtes en plastique transparentes aux UV ( boîtes polystyrène cristal 50x40x6 $cm^3$) et exposées aux UV (SUNTEST CPS Heraeus) pendant 1H. Les mesures colorimétriques ont été effectuées à l'aide d'un colorimètre Minolta CM1000 : une première mesure a été relevée juste avant l'exposition aux UV (T0) et une deuxième après une heure d'exposition aux UV (T1H).

Les résultats sont exprimés dans le système (L, a, b) dans lequel L représente la luminance, a représente l'axe rouge-vert (-a = vert, +a = rouge) et b représente l'axe jaune-bleu (-b =bleu, +b =jaune).

Pour l'évaluation du photobleuissement, on s'intéresse au $\Delta b$, qui mesure la variation de la couleur bleue, à $\Delta L$, qui mesure le noircissement de la composition et à $\Delta E$, qui mesure la variation de couleur totale. Plus précisément, $\Delta b$ et $\Delta L$ sont définis par $\Delta b = b_{T1H} - b_{T0}$ et $\Delta L = L_{T1H} - L_{T0}$. Plus $\Delta b$ est faible, plus la protection contre le photobleuissement est efficace.

$\Delta E$ est calculé à partir des variations $\Delta L$, $\Delta a$ et $\Delta b$ selon la formule suivante :

$$\Delta E = \sqrt{(\Delta L)^2 + (\Delta a)^2 + (\Delta b)^2}$$

Les résultats sont consignés dans le tableau (I) suivant :

Tableau (I)

| Formule | x% | $\Delta L$ | $\Delta b$ | $\Delta E$ |
|---|---|---|---|---|
| A (comparatif) | 0 | 10,7 | 7,7 | 13,60 |
| B | 0,2 | 8,0 | 4,45 | 9,55 |
| C | 0,5 | 3,15 | 0,27 | 3,30 |
| D | 1 | 4,6 | 0,95 | 4,80 |
| E | 2 | 2,75 | 0,14 | 2,80 |

Ces résultats montrent clairement que l'introduction de microsphères creuses déformables d'un copolymère expansé de chlorure de vinylidène et d'acrylonitrile ou de chlorure de vinylidène, d'acrylonitrile et de méthacrylate au sein d'une composition anhydre comportant un oxyde de titane diminue significativement le photobleuissement dû à la présence dudit oxyde de titane.

## EXEMPLE 2 :

On a repris les cinq compositions anhydres A, B, C, D et E de l'exemple 1 et on a évalué le blanchiment observé lors de l'application de ces compositions sur la peau.

Pour cette évaluation du blanchiment, des tests ont été réalisés sur cinq modèles. L'intensité du blanchiment (Ib) est mesurée selon une échelle graduée de 0 à 5. La valeur 0 correspond à une composition très blanchissante tandis que la valeur 5 correspond à une composition très peu blanchissante.

Les résultats sont consignés dans le tableau (I) suivant :

Tableau (I)

| Formule | x% | Ib |
|---|---|---|
| A (comparatif) | 0 | 2 |
| B | 0,2 | 2,9 |
| C | 0,5 | 2,8 |
| D | 1 | 3,6 |
| E | 2 | 3,7 |

Ces résultats montrent clairement que l'introduction de microsphères creuses déformables d'un copolymère expansé de chlorure de vinylidène et d'acrylonitrile ou de chlorure de vinylidène, d'acrylonitrile et de méthacrylate au sein d'une composition comportant un oxyde de titane diminue significativement le blanchiment de la composition lors de son application sur la peau.

**EXEMPLE 3 :**

On a réalisé trois émulsions huile-dans-eau (H/E) contenant un certain pourcentage x d'Expancel et 20% d'oxyde de titane, et de formule suivante (les quantités sont données en % de poids par rapport au poids total de la composition) :

Phase A :

| | |
|---|---|
| - microsphères expansées de copolymère chlorure de vinylidène / acrylonitrile / méthacrylate de méthyle contenant de l'isobutane vendues sous la dénomination commerciale « Expancel 551 DE » par Nobel Casto | x% |
| - alcool cétylstéarylique oxyéthyléné 12 OE vendu sous la dénomination commerciale « Eumulgin B1 » par Henkel (émulsionnant) | 3,3% |
| - monostéarate de glycérol vendu sous la dénomination commerciale « Tegin 90 » par Goldschmidt (émulsionnant) | 1,7% |
| - di-caprylyl éther vendu sous la dénomination commerciale « Cetiol OE » par Henkel (huile) | 17,5% |
| - cyclohexa diméthylsiloxane vendu sous la dénomination commerciale « Dow Corning 246 Fluid » par Dow Corning (huile) | 5,6% |

Phase B :

| | |
|---|---|
| - oxyde de titane vendu sous la dénomination commerciale « MT 100T » par Tayca (pigment) | 20% |

Phase C :

| | |
|---|---|
| - hydroxyéthylcellulose vendue sous la dénomination commerciale « Natrosol 250 HHR » par Aqualon (épaississant) | 0,5% |
| - gomme de xanthane vendue sous la dénomination commerciale « Keltrol T » par Kelco (épaississant) | 0,4% |

Phase D :

| | |
|---|---|
| - hydratants | 15% |
| - conservateurs | qs |

Phase E :

| | |
|---|---|
| - eau purifiée | qsp 100% |

Les émulsions ont été réalisées de la manière suivante : on a chauffé la phase A à 90°C. On lui a ensuite ajouté la moitié de la phase E et la phase D préalablement chauffées à 90°C et on a mélangé sous agitation pour réaliser l'émulsion. On a ajouté la phase B sous agitation, puis la phase C préalablement dispersée dans l'autre moitié de la phase E. On a continué le refroidissement jusqu'à température ambiante.

On a ainsi réalisé trois émulsions H/E différentes 1, 2 et 3 en faisant varier la teneur x de 0 à 1%.

On a également réalisé trois émulsions eau-dans-huile (E/H) contenant un certain pourcentage x d'Expancel et 20% d'oxyde de titane, et de formule suivante (les quantités sont données en % de poids par rapport au poids total de la composition) :

Phase A1 :

| | |
|---|---|
| - microsphères expansées de copolymère chlorure de vinylidène / acrylonitrile / méthacrylate de méthyle contenant de l'isobutane vendues sous la dénomination commerciale « Expancel 551 DE » par Nobel Casto | x% |
| - poly diméthyl / méthyl cétyl / méthyl siloxane oxyéthyléné vendu sous la dénomination commerciale « Abil EM 90 D » par Goldschmidt (émulsionnant) | 2% |
| - néopentanoate d'isoarachidyle vendu sous la dénomination commerciale « Elefac I205 » par Bernel (huile) | 10% |
| - 2,2,4,4,6,6,8 heptaméthylnonane vendu sous la dénomination commerciale « Isohexadécane » par Bayer (huile) | 10% |
| - cire d'abeille naturelle de Barlocher | 1,5% |
| - « Cero 20 EG 626 » de Barlocher (cire - épaississant) | 1% |

Phase A2 :

| | |
|---|---|
| - oxyde de titane vendu sous la dénomination commerciale « MT 100T » par Tayca (pigment) | 20% |

Phase B :

| | |
|---|---|
| - chlorure de sodium | 2% |
| - sulfate de magnésium | 0,7% |
| - hydratants | 12% |

Phase C :

| | |
|---|---|
| - eau purifiée | qsp 100% |

Ces émulsions ont été réalisées de la manière suivante :on a chauffé la phase A1 à 80°C puis on a ajouté la phase A2 sous agitation. On a ensuite chauffé les phases C et B à 80°C. Puis on a mélangé les phase A1, A2, C et B sous agitation et on a continué le refroidissement jusqu'à température ambiante.

On a ainsi réalisé trois émulsions E/H différentes 4, 5 et 6 en faisant varier la teneur x de 0 à 1%.

Le protocole d'évaluation du photobleuissement est le même que pour l'exemple 1.

Les résultats sont consignés dans le tableau (II) suivant, où $\Delta b$, $\Delta L$ et $\Delta E$ ont les mêmes significations que pour l'exemple 1 :

Tableau (II)

| Emulsion | x% | ΔL | Δb | ΔE |
|---|---|---|---|---|
| 1 (H/E) comparative | 0 | 24,05 | 16,55 | 29,70 |
| 2 (H/E) invention | 0,5 | 15,50 | 9,65 | 18,75 |
| 3 (H/E) invention | 1 | 12,85 | 8,85 | 16,10 |
| 4 (E/H) comparative | 0 | 22,80 | 18,00 | 29,65 |
| 5 (E/H) invention | 0,5 | 18,30 | 13,65 | 23,45 |
| 6 (E/H) invention | 1 | 16,60 | 12,65 | 21,55 |

Ces résultats montrent clairement que l'introduction de microsphères creuses déformables d'un copolymère expansé de chlorure de vinylidène et d'acrylonitrile ou de chlorure de vinylidène, d'acrylonitrile et de méthacrylate au sein d'une émulsion H/E ou E/H comportant un oxyde de titane diminue significativement le photobleuissement dû à la présence dudit oxyde de titane.

EXEMPLE 4 :

La composition suivante donne un exemple concret d'une crème solaire anhydre haute protection conforme à l'invention. Les quantités sont exprimées en % de poids par rapport au poids total de la composition :

Phase A :

| | |
|---|---|
| - alcool butylique oxypropyléné 14 OP vendu sous la dénomination commerciale « Ucon Fluid AP » par Amerchol (émulsionnant) | 12% |
| - palmitate d'isopropyle vendu sous la dénomination commerciale « Estol 1517 » par Unichema | 12% |
| - huile de ricin hydrogénée vendue sous la dénomination commerciale « Cutina HR » par Henkel | 7,5% |
| - monostéarate de glycérol vendu sous la dénomination commerciale « Tegin 90 » par Goldschmidt | 0,25% |

Phase B :

| | |
|---|---|
| - oxyde de titane vendu sous la dénomination commerciale « MT 100T » par Tayca | 20% |
| - silicate de magnésium vendu sous la dénomination commerciale « Talc Luzenac 15 M00 » par Luzenac | 5% |
| - microsphères expansées de copolymère chlorure de vinylidène / acrylonitrile / méthacrylate de méthyle contenant de l'isobutane vendues sous la dénomination commerciale « Expancel 551 DE » par Nobel Casto | 0,5% |
| - polydécène hydrogéné (PM 549) vendu sous la dénomination commerciale « Silkflo S 366 NF » par Albemarle | qsp 100% |

Cette crème a été réalisée de la façon suivante : on a chauffé la phase A à 90°C afin de l'homogénéiser. Puis on a préparé la pâte pigmentaire en mélangeant l'oxyde de titane au polydécène hydrogéné. On a ajouté à ce mélange le talc puis l'Expancel. La phase B ainsi réalisée a été chauffée à 90°C. Puis on a mélangé les deux phases A et B au Rayneri. Une fois le mélange pris en masse, on a de nouveau chauffé à 90°C. Puis on a turbiné le mélange au Turrax pendant 10 minutes. On a ensuite laissé refroidir la crème à température ambiante sous agitation Rayneri.

Cette crème est très faiblement sujette au photobleuissement et blanchit très faiblement lorsqu'on l'applique sur la peau.

**Revendications**

1. Utilisation de particules creuses déformables, dans une composition cosmétique et/ou dermatologique contenant des pigments d'oxyde de titane, pour diminuer ou éliminer le photobleuissement dû à la présence desdits pigments et/ou le blanchiment lors de l'application de ladite composition sur la peau, ces particules creuses présentant une granulométrie allant de 1 μm à 250 μm, et étant formées d'un copolymère de chlorure de vinylidène, d'acrylonitrile et de (méth)acrylate de méthyle, expansé.

2. Utilisation selon la revendication 1, caractérisée en ce que les particules creuses ont une granulométrie inférieure à 100 μm.

3. Utilisation selon la revendication 1 ou 2, caractérisée en ce que les particules creuses ont une granulométrie allant de 10 μm à 60 μm.

4. Utilisation selon les revendications précédentes, caractérisée en ce que les particules creuses ont une masse volumique allant de 15 kg/m$^3$ à 200 kg/m$^3$.

5. Utilisation selon la revendication 4, caractérisée en ce que les particules creuses ont une masse volumique allant de 40 kg/m$^3$ à 100 kg/m$^3$.

6. Utilisation selon l'une des revendications précédentes, caractérisée en ce que les particules creuses représentent de 0,1 % à 10 % en poids du poids total de la composition.

7. Utilisation selon la revendication 6, caractérisée en ce que les particules creuses représentent de 0,3 % à 5 % en poids du poids total de la composition.

8. Utilisation selon l'une des revendications précédentes, caractérisée en ce que ladite composition contient au moins 5% en poids d'oxyde de titane, par rapport au poids total de la composition.

9. Utilisation selon la revendication 8, caractérisée en ce que ladite composition contient au moins 10% en poids d'oxyde de titane, par rapport au poids total de la composition.

10. Utilisation selon la revendication 9, caractérisée en ce que ladite composition contient au moins 15% en poids d'oxyde de titane, par rapport au poids total de la composition.

11. Utilisation selon l'une des revendications précédentes, caractérisée en ce que ladite composition contient, en outre, au moins un adjuvant choisi parmi les épaississants, les émulsionnants, les gélifiants, les actifs hydrophiles, les actifs lipophiles, les parfums, les conservateurs, les solvants, les matières colorantes.

**Claims**

1. Use of hollow, deformable particles, in a cosmetic and/or dermatological composition containing titanium oxide pigments, for decreasing or eliminating the photoblueing due to the presence of the said pigments and/or the whitening when the said composition is applied to the skin, these hollow particles having a particle size ranging from 1 μm to 250 μm, and being formed of an expanded copolymer of vinylidene chloride, acrylonitrile and methyl (meth)acrylate.

2. Use according to Claim 1, characterized in that the hollow particles have a particle size of less than 100 μm.

3. Use according to Claim 1 or 2, characterized in that the hollow particles have a particle size ranging from 10 μm to 60 μm.

4. Use according to the preceding claims, characterized in that the hollow particles have a density ranging from 15 kg/m$^3$ to 200 kg/m$^3$.

5. Use according to Claim 4, characterized in that the hollow particles have a density ranging from 40 kg/m$^3$ to 100 kg/m$^3$.

6. Use according to one of the preceding claims, characterized in that the hollow particles represent from 0.1% to 10% by weight relative to the total weight of the composition.

7. Use according to Claim 6, characterized in that the hollow particles represent from 0.3% to 5% by weight relative to the total weight of the composition.

8. Use according to one of the preceding claims, characterized in that the said composition contains at least 5% by weight of titanium oxide relative to the total weight of the composition.

9. Use according to Claim 8, characterized in that the said composition contains at least 10% by weight of titanium oxide relative to the total weight of the composition.

10. Use according to Claim 9, characterized in that the said composition contains at least 15% by weight of titanium oxide relative to the total weight of the composition.

11. Use according to one of the preceding claims, characterized in that the said composition also contains at least one adjuvant chosen from thickeners, emulsifiers, gelling agents, hydrophilic active agents, lipophilic active agents, fragrances, preserving agents, solvents and dyestuffs.

**Patentansprüche**

1. Verwendung von verformbaren Hohlpartikeln in einer kosmetischen und/oder dermatologischen Zusammensetzung, die Pigmente von Titandioxid enthält, zur Minderung oder Verhinderung der Photobläuung, die auf die Anwesenheit dieser Pigmente zurückzuführen ist, und/oder der Weißfärbung beim Auftragen der Zusammensetzung auf die Haut, wobei diese Hohlpartikel eine Teilchengröße von 1 bis 250 µm aufweisen und aus einem expandierten Copolymer von Vinylidenchlorid, Acrylnitril und Methyl(meth)acrylat bestehen.

2. Verwendung nach Anspruch 1, dadurch gekennzeichnet, daß die Hohlpartikel eine Teilchengröße von unter 100 µm aufweisen.

3. Verwendung nach Anspruch 1 oder 2, dadurch gekennzeichnet, daß die Hohlpartikel eine Teilchengröße von 10 bis 60 µm aufweisen.

4. Verwendung nach den vorhergehenden Ansprüchen, dadurch gekennzeichnet, daß die Hohlpartikel eine Dichte von 15 bis 200 kg/m$^3$ aufweisen.

5. Verwendung nach Anspruch 4, dadurch gekennzeichnet, daß die Hohlpartikel eine Dichte von 40 bis 100 kg/m$^3$ aufweisen.

6. Verwendung nach einem der vorhergehenden Ansprüche, dadurch gekennzeichnet, daß die Hohlpartikel 0,1 bis 10 Gew.-% des Gesamtgewichts der Zusammensetzung ausmachen.

7. Verwendung nach Anspruch 6, dadurch gekennzeichnet, daß die Hohlpartikel 0,3 bis 5 Gew.-% des Gesamtgewichts der Zusammensetzung ausmachen.

8. Verwendung nach einem der vorhergehenden Ansprüche, dadurch gekennzeichnet, daß die Zusammensetzung mindestens 5 Gew.-% Titandioxid, bezogen auf das Gesamtgewicht der Zusammensetzung, enthält.

9. Verwendung nach Anspruch 8, dadurch gekennzeichnet, daß die Zusammensetzung mindestens 10 Gew.-% Titandioxid, bezogen auf das Gesamtgewicht der Zusammensetzung, enthält.

10. Verwendung nach Anspruch 9, dadurch gekennzeichnet, daß die Zusammensetzung mindestens 15 Gew.-% Titandioxid, bezogen auf das Gesamtgewicht der Zusammensetzung, enthält.

11. Verwendung nach einem der vorhergehenden Ansprüche, dadurch gekennzeichnet, daß die Zusammensetzung ferner mindestens einen Hilfsstoff enthält, der unter den Verdickungsmitteln, den Emulgatoren, den Gelbildnern, den hydrophilen Wirkstoffen, den lipophilen Wirkstoffen, den Parfums, den Konservierungsmitteln, den Lösungsmitteln und den Färbemitteln ausgewählt ist.